(19) Europäisches Patentamt
European Patent Office
Office européen des brevets

(11) Publication number: 0 352 977
A2

(12) EUROPEAN PATENT APPLICATION

(21) Application number: 89307407.0

(51) Int. Cl.4: C07D 333/48

(22) Date of filing: 20.07.89

(30) Priority: 28.07.88 US 225408

(43) Date of publication of application:
31.01.90 Bulletin 90/05

(84) Designated Contracting States:
AT BE CH DE ES FR GB GR IT LI LU NL SE

(71) Applicant: PFIZER INC.
235 East 42nd Street
New York, N.Y. 10017(US)

(72) Inventor: Quallich, George J.
349, Norwich Westerly Road
North Stonington Connecticut(US)

(74) Representative: Moore, James William, Dr.
Pfizer Limited Ramsgate Road
Sandwich Kent CT13 9NJ(GB)

# (54) Stereoselective oxidation process.

(57) Oxidation of certain 3-substituted tetrahydrothiophenes with potassium peroxymonosulfate to the corresponding sulfoxides proceeds with a high degree of stereoselectivity. In the sulfoxide product, the isomer in which the 3-substituent and the sulfoxide oxygen are trans predominates over the corresponding cis-isomer by a factor of at least 10:1. The 3-substituent is a leaving group, such as I, Br or a sulfonyloxy group. The products of the oxidation process are intermediates to penem antibiotics known from U.S. Patent No. 4,619,924.

EP 0 352 977 A2

## STEREOSELECTIVE OXIDATION PROCESS

This invention relates to an improved process for preparing certain chemical compounds, useful as intermediates to penem antibiotics. More particularly it relates to a process for the oxidation of certain tetrahydrothiophenes having a leaving group X at the 3-position, to give the corresponding sulfoxides. The process uses potassium peroxymonosulfate as the oxidant, and it proceeds stereoselectively, to produce a high preponderance of the isomer in which the sulfoxide oxygen and the X substituent are in a trans relationship towards each other.

Potassium peroxymonosulfate is known to oxidize sulfides to sulfoxides. Trost et al, Tetrahedron Letters, Vol. 22, pp. 1287-90 (1981). See also U.S. Patent No. 4,316,842, issued February 23,1982. Other oxidants (e.g., m-chloroperbenzoic acid) can be used for the present process, but they produce only low stereoselectivity, with the result that difficult separation procedures (e.g., chromatography) are needed to obtain substantially pure trans-isomer. By contrast, the present process produces the sulfoxide product having a trans to cis ratio of at least 10:1, such that substantially pure trans-isomer is obtainable simply by recrystallization.

This invention provides a chemical process for preparing a sulfoxide compound of the formula

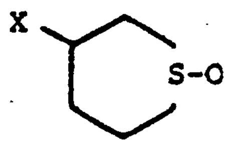

---(I)

in which the X group and the sulfoxide oxygen atom are in a trans relationship to each other, which comprises reacting the corresponding sulfide of the formula

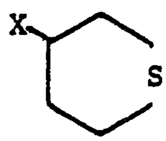

---(II)

with potassium peroxymonosulfate. X is a leaving group, such as I, Br, Cl, sulfonyloxy of the formula $R-SO_2-O$ or acyloxy of the formula R-CO-O, where R is $C_1$-$C_4$ alkyl, trifluoromethyl, phenyl, 4-nitrophenyl or 4-tolyl.

Preferred values for X are I, Br and $R-SO_2-O$, where R is as defined above. Especially preferred for X is 4-tolylsulfonyloxy.

The process of the invention is carried out in a reaction-inert solvent, such as acetone, aqueous acetone, methanol or aqueous methanol.

The sulfoxide products of this invention are useful as intermediates to penem antibiotics of the formula

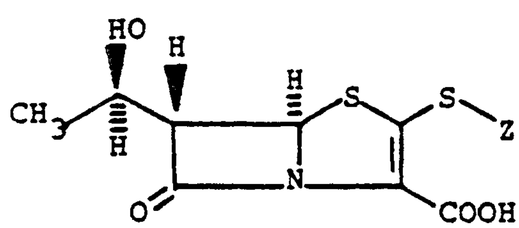

---(III)

wherein Z is the group of the formula

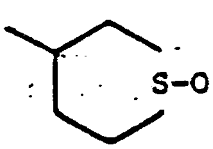

---(IV)

in which the sulfoxide oxygen and the bond at the 3-position of the tetrahydrothiophene ring are cis to each other.

The process of this invention is normally carried out simply by contacting the sulfide of the formula II with an alkali metal peroxymonosulfate, preferably potassium peroxymonosulfate, in a reaction-inert solvent, until conversion to sulfoxide is essentially complete.

A reaction-inert solvent is one which does not adversely interact with either the starting materials or the product. Additionally, it is usually preferable that at least one of the starting materials, and also the product, are soluble to a significant extent in the solvent. Yet further, the solvent should be chosen such that the sulfoxide can be readily isolated. This usually means that the solvent is miscible with water and/or sufficiently volatile to be removable by evaporation or distillation. With these factors in mind, typical solvents which can be used for the process of this invention are lower alkanols (e.g., $C_1$-$C_4$ alkanols, such as methanol and ethanol), di(lower alkyl) ketones (e.g., di($C_1$-$C_4$ alkyl) ketones, such as acetone and methyl isobutyl ketone), di(lower alkyl) ethers (e.g., di($C_1$-$C_4$ alkyl) ethers, such as diethyl ether), tetrahydrofuran, dioxan, lower alkanoic acids (e.g., acetic acid), acetonitrile, water and mixtures of these solvents. Methanol and acetone, with or without added water, are commonly used.

The process of this invention proceeds at a convenient rate at temperatures from about -10 to about 30° C, and preferably from 0 to 20° C. A particularly convenient way of operating the process is to combine the starting sulfide and oxidant, in an appropriate solvent, at about 0° C, and then allow the reaction mixture to warm slowly to room temperature.

The ratio of reactants must be carefully controlled in order to obtain high yields of sulfoxide. In order to achieve substantially complete conversion of sulfide to sulfoxide, and ensure a trans to cis ratio of at least 10:1, from about 1.0 to about 1.5 molar equivalents of potassium peroxymonosulfate should be used. The preferred amount of potassium peroxymonosulfate is about 1.2 molar equivalents. Moreover, in order to maximize the stereoselectivity, it is preferable to combine all of the starting materials at the outset of the reaction.

The time course of the reaction varies according to the reaction temperature and the concentration of the reaction mixture in the expected manner--i.e., the reaction proceeds more rapidly at higher temperatures and at higher concentrations. Thus, reaction times vary according to these factors. In general, it is convenient to monitor the progress of the reaction by thin-layer chromatography. However, at about 0° C, and at concentrations of reactants of about 0.1 molar, reaction times of about 1 to 2 hours are commonly used.

The sulfoxide product of the formula I can be isolated by conventional methods. For example, if a water-miscible solvent has been used for the oxidation reaction, it is convenient simply to dilute the reaction mixture with water and extract the sulfoxide product into a water-immiscible, volatile organic solvent, such as chloroform or dichloromethane. Evaporation of the solvent then affords the crude sulfoxide I. Alternatively, if a water-immiscible organic solvent has been used for the oxidation reaction, the reaction medium can simply be washed with water. Evaporation of the solvent then affords the crude sulfoxide I. Yet further, if a volatile solvent has been used for the oxidation reaction, that solvent can be removed by evaporation, and the residue is partitioned between an aqueous phase and a volatile, water-immiscible organic solvent. The layers are then separated and the organic phase is evaporated to give the crude sulfoxide I.

The crude sulfoxide is usually pure enough for use as a chemical intermediate. However, it can be purified further by conventional means, such as chromatography or recrystallization, if desired.

The sulfide of the formula II can exist in either of two enantiomeric (optically active) forms, or mixtures thereof (e.g., as a 1:1 racemic mixture). Either of the optically-active forms, or a mixture thereof, can serve as substrate for the present oxidation process. Also, the process always adds the oxygen atom to sulfur without affecting the stereochemistry of the substituent X, and in a manner in which the isomer with the oxygen atom trans to the X substituent predominates in the sulfoxide product.

As indicated hereinbefore, the sulfoxides produced by the process of this invention are useful for preparing penem antibiotics of the formula III above. Methods for converting the sulfoxides produced by the process of this invention into the penem antibiotics of formula III are described in United States Patent No. 4,619,924. See also pending international application PCT/US87/01114 and pending U.S. application Serial No. 07/183,102.

The starting sulfides of the formula II are either known compounds, which can be prepared by the known method, or they are analogs of known compounds, which can be prepared by methods analogous to those used for the known compounds. See, U.S. Patent No. 4,619,924. See also pending international application PCT/US87/01114 and pending U.S. application Serial No. 07/183,102.

The following examples are provided solely for the purpose of further illustration. The oxidant used was a mixture having the following composition:

$2KHSO_5.KHSO_4.K_2SO_4$.

## EXAMPLE 1

### 3-(4-Tolysulfonyloxy)tetrahydrothiophene 1-Oxide, Trans Isomer, Racemic

At 0 °C, a solution of 2.80 g (9.12 mmole) of potassium peroxymonosulfate in 40 ml of distilled water was added to a solution of 2.00 g (7.75 mmole) of 3-(4-tolylsulfonyloxy)tetrahydrothiophene in 40 ml of acetone, with stirring. Stirring was continued for 40 minutes, without external cooling, and then 80 ml of water was added to the cloudy reaction mixture. Stirring was continued until a clear solution was obtained (ca. 2 minutes). The clear solution was extracted with dichloromethane, and the extracts were dried ($MgSO_4$) and evaporated in vacuo. This produced an oil. To the oil was added a seed crystal of the title compound from a previous preparation. This caused the oil to crystallize, giving 2.10 g of crude product. This material contained 90% of the title compound, 5% of the cis-isomer and 5% other impurities.

The crude product was recrystallized from toluene to give 1.08 g (51% yield) of a first crop, which was 98% pure and had a trans to cis ratio of 44:1. A second crop, 0.51 g (24% yield), which was 88% pure and had a trans to cisratio of 10:1, was also recovered.

## EXAMPLE 2

### Solvent Study

A series of reactions were run in order to assess the effect of the solvent on the trans/cis isomer ratio for the oxidation process of this invention.

Method.

At 0 °C, a solution of 156 mg (0.51 mmole) of potassium peroxymonosulfate in 2 ml of the test solvent was added, in one portion, to a solution of 100 mg (0.39 mmole) of racemic 3-(4-tolylsulfonyloxy)-tetrahydrothiophene in 2 ml of the test solvent, with stirring. Stirring was continued and the reaction mixture was allowed to warm to room temperature. After 45 minutes, the reaction mixture was diluted with 10 ml of water and stirred until a clear solution was obtained. The product was extracted into chloroform, which was dried and evaporated to give the product. The product was analyzed by high-pressure liquid chromatography. The results are shown in Table 1. The entries under "% Cis" and "% Trans" indicate the absolute percentage in the crude product.

TABLE I

| Test Solvent | % Cis | % Trans | Trans/Cis Ratio |
|---|---|---|---|
| Methanol | 2.53 | 58.00 | 15:1[1] |
| Isopropanol | 6.36 | 93.64 | 15:1 |
| t-Butanol | 7.48 | 82.38 | 11:1 |
| Acetone | 6.23 | 93.77 | 15:1 |
| Acetic Acid | 8.51 | 91.49 | 11:1 |
| Acetonitrile | 6.57 | 91.67 | 14:1 |
| Acetonitrile | 6.22 | 90.76 | 15:1 |
| Tetrahydrofuran | 5.19 | 94.81 | 18:1 |
| Tetrahydrofuran | 2.46 | 83.77 | 34:1 |

[1] The reaction product contained ca. 40% of unreacted sulfide

## EXAMPLE 3

### 3-(Trifluoromethylsulfonyloxy)tetrahydrothiophene 1-Oxide, Trans Isomer, Racemic

3-(Trifluoromethylsulfonyloxy)tetrahydrothiophene (969 mg, 4.1 mmole) was dissolved in 10 ml of acetone and cooled to -5° C. The potassium peroxymonosulfate (1.51 g, 4.92 mmole) dissolved in 10 ml of water was added over 15 min. keeping the temperature below 0° C. The solution was stirred for 30 min. and then quenched with 2 ml. of 10% aqueous sodium bisulfite. After concentrating the reaction to one-half the original volume under vacuum, the contents were extracted with ethyl acetate, washed with brine, and dried with magnesium sulfate. Removal of the solvent under vacuum provided 369 mg (35%) of product as an oil. By high field proton nuclear magnetic resonance spectroscopy, the product had a trans to cis ratio of 10:1.

## EXAMPLE 4

### 3-(Methylsulfonyloxy)tetrahydrothiophene 1-Oxide, Trans Isomer, Racemic

3-Methylsulfonyloxy)tetrahydrothiophene (600 mg, 3.3 mmoles) was dissolved in acetone (6 ml) and cooled to 0° C. Potassium peroxymonosulfate (1.23 g, 3.96 mmoles) dissolved in 6 ml of water was added over 1 min and the reaction stirred for 15 min. The reaction volume was reduced to one-half volume under vacuum. The aqueous layer was extracted with chloroform, the organic phase washed with brine, and dried over magnesium sulfate. After removal of the solvent under vacuum, a viscous oil was obtained which crystallized on standing to afford a white solid (538 mg, 82%). High field proton and carbon nuclear magnetic resonance spectroscopy showed that the trans to cis ratio was greater than 10:1.

## EXAMPLE 5

### 3-(4-Nitrophenylsulfonyloxy)tetrahydrothiophene 1-Oxide, Trans Isomer, Racemic

3-(4-Nitrophenylsulfonyloxy)tetrahydrothiophene (600 mg, 2.33 mmole) was dissolved in 6 ml of acetone and cooled to -5° C. An aqueous solution of potassium peroxymonosulfate (860 mg, 2.8 mmoles in 6 ml of water) was added dropwise keeping the temperature ca. 0° C. The oxidant addition took 15 min. and the reaction was stirred for another 30 min. Aqueous sodium bisulfite (2 ml of 10%) was employed to quench the reaction and the volume was reduced to small volume under vacuum. Ethyl acetate/water (1:1) was added followed by separation of the phases. The organic phase was washed with brine, dried over magnesium sulfate, and evaporated under vacuum. The crude product was crystallized from toluene to provide 494 mg (78%) of the desired sulfoxide with the trans to cis ratio of 95:5 by high field proton nuclear magnetic resonance spectroscopy.

## EXAMPLE 6

### 3-Bromotetrahydrothiophene, 1-Oxide, Trans Isomer, Optically Active

(3S)-3-Bromotetrahydrothiophene (24 g, 0.143 mole) was dissolved in 846 ml of acetone and cooled to -30° C. Aqueous potassium peroxymonosulfate (44 g, .143 mole in 340 ml of water) was added over 40 min., maintaining the temperature at -25 to -30° C. After stirring 10 min., aqueous peroxymonosulfate was added (4.4 g, 0.0143 mole, in 34 ml of water) and stirring continued for 10 min. Then another charge of aqueous potassium peroxymonosulfate (4.4 g, 0.0143 mole in 34 ml of water) was made. Ten minutes later, the reaction was quenched with aqueous sodium bisulfite (600 mg in 60 ml of water). The reaction was concentrated under vacuum, and extracted with methylene chloride three times (600 ml, 400 ml, and 200 ml). The combined organic layers were washed with brine (200 ml) and dried over magnesium sulfate (50 g). The methylene chloride was displaced with ethyl acetate, and then hexanes, and cooled to 0° C as the product precipitated out of solution. The product sulfoxide was isolated by vacuum filtration to afford 19.1 g (73% yield) of product, mp 64-67° C (95% pure). The mother liquor was evaporated to an oil (6.4 g) and then it was recrystallized from methylene chloride/hexanes to yield another 4.41 g (16% yield) of product. The latter material was ca. 95% pure and contained ca. 4% of cis isomer.

## EXAMPLE 7

### 3-Iodotetrahydrothiophene, 1-Oxide, Trans Isomer, Racemic

3-Iodotetrahydrothiophene (3.75 g, 17.5 mmoles) was dissolved in 30 ml of acetone and cooled to 0° C. Aqueous potassium peroxymonosulfate (6.46 g, 21 mmoles) was added over 1 hr. and the reaction stirred for an additional 15 min. Aqueous sodium bisulfite (10 ml of 10%) was added and the reaction reduced to one-half volume under vacuum. Ethyl acetate/water (1:1) was added, and the phases were separated. The organic phase was washed with brine, dried with sodium sulfate, and the solvent removed under vacuum yielding a yellow-white solid. This solid was dissolved in diethyl ether, and ispropyl ether was added until a cloudy solution resulted. After stirring 20 min., the solid was filtered off, and dried to give 2.19 g (54%) of the desired sulfoxide with a trans to cis ratio of 95:5.

## Claims

1. A process for preparing a sulfoxide compound of the formula

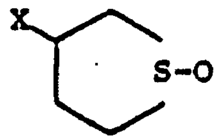

in which the X group and the sulfoxide oxygen atom are in a trans relationship to each other;
wherein X is I, Br or R-$SO_2$-O, wherein R is $C_1$-$C_4$ alkyl, trifluoromethyl, phenyl, 4-nitrophenyl or 4-tolyl; said process comprising reacting the corresponding sulfide of the formula

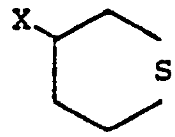

with from 1.0 to 1.5 molar equivalents of potassium peroxymonosulfate, in a reaction-inert solvent, at a temperature from -10 to 30 °C, until conversion from sulfide to sulfoxide is substantially complete.

2. The process according to claim 1, wherein X is R-$SO_2$-O.

3. The process according to claim 2, wherein said reaction-inert solvent is selected from $C_1$-$C_4$ alkanols, di($C_1$-$C_4$ alkyl) ketones, di($C_2$-$C_4$ alkyl) ethers, tetrahydrofuran, dioxan, acetic acid, acetonitrile, water and mixtures thereof.

4. The process according to claim 3, wherein said reaction-inert solvent is aqueous methanol or aqueous acetone.

5. The process according to claim 4, wherein R is methyl.

6. The process according to claim 4, wherein R is 4-tolyl.

7. The process according to claim 6, wherein said process is carried out using 1.2 molar equivalents of potassium peroxymonosulfate in aqueous acetone at 0 °C.